# FASCICULE DE BREVET EUROPEEN

(11) **EP 2 011 477 B1**
(45) Date de publication et mention de la délivrance du brevet: **06.03.2013**
(21) Numéro de dépôt: 08158237.1
(22) Date de dépôt: 13.06.2008
(51) Int. Cl.: A61K 8/44, A61K 8/46, A61K 8/60, A61K 8/81, A61K 8/891, A61Q 5/12, A61Q 5/02, A61K 8/36

(54) **Compositions cosmétiques détergentes comprenant quatre tensioactifs, un polymère cationique et un agent bénéfique et utilisation**
Kosmetische Reinigungszusammensetzungen enthaltend vier Tenside, ein kationisches Polymer und ein Pflegemittel und deren Verwendung
Detergent cosmetic compositions comprising four surfactants, a cationic polymer and a beneficial agent, and use thereof

(30) Priorité: 29.06.2007 FR 0756160
(43) Date de publication de la demande: 07.01.2009
(73) Titulaire: L'Oréal, 75008 Paris (FR)
(72) Inventeur: Paul, Laurence, 95320 Saint-Leu-la-Forêt (FR); Giroud, Franck, 73390 Chamoux sur Gelon (FR); Samain, Henri, 91570 Bièvres (FR)
(74) Mandataire: Le Blainvaux Bellegarde, Françoise

(56) Documents cités:
- EP-A- 0 603 078
- EP-A- 0 974 335
- EP-A- 1 321 124
- EP-A- 1 661 976
- EP-A- 1 726 293
- WO-A-97/33561
- DE-A1- 4 302 314
- DE-A1- 4 409 189
- DE-A1- 19 723 763
- FR-A- 2 718 961

## Description

La présente invention concerne de nouvelles compositions cosmétiques détergentes des matières kératiniques, en particulier des fibres kératiniques humaines telles que les cheveux, comprenant, dans un support aqueux cosmétiquement acceptable, au moins un tensioactif anionique sulfate ou sulfonate, au moins un agent tensioactif anionique carboxylique différent du précédent, au moins un tensioactif amphotère et au moins un tensioactif non ionique de type alkylpolyglycoside. L'invention concerne aussi l'utilisation desdites compositions dans l'application cosmétique susmentionnée.

Il est bien connu que les cheveux sont sensibilisés ou fragilisés à des degrés divers par l'action des agents atmosphériques et notamment de la lumière, l'eau et l'humidité ainsi que par des traitements mécaniques ou chimiques répétés tels que le brossage, le peignage, le lavage, les décolorations, les permanentes, les défrisages et/ou les teintures. De nombreuses publications divulguent que la lumière naturelle détruit certains aminoacides des cheveux. Ces agressions altérant la fibre capillaire, elles en diminuent les propriétés mécaniques comme la résistance à la traction, la charge à la rupture et l'élasticité, ou leur résistance au gonflement dans un milieu aqueux. Les cheveux sont ternes, rêches et cassants. Les cheveux sont difficiles à démêler et à coiffer.

On sait également que notamment la lumière et les agents de lavage ont tendance à agresser la couleur naturelle des cheveux ainsi que la couleur artificielle des cheveux teints. La couleur des cheveux s'affaiblit peu à peu ou vire vers des nuances peu esthétiques ou indésirables.

On recherche depuis de nombreuses années, dans l'industrie cosmétique des substances permettant de protéger les cheveux des dégradations engendrées par les agressions atmosphériques, telles que la lumière et la chaleur et les traitements. En particulier, on recherche des produits améliorant les propriétés cosmétiques (notamment le démêlage, la douceur, le lissage, la brillance...), des produits protégeant la couleur des fibres kératiniques colorées naturellement ou teintes artificiellement et préservant ou renforçant les propriétés mécaniques intrinsèques des fibres kératiniques (la résistance à la traction, la charge à la rupture et l'élasticité, ou leur résistance au gonflement dans un milieu aqueux).

Par ailleurs, la peau peut être également altérée à des degrés divers par l'action des agents atmosphériques ainsi que par l'action répétée de produits détergents. Les tissus cutanés peuvent être brûlés, la peau devient sèche et rêche et perd de son élasticité naturelle. Une apparition de pellicules, d'un excès de séborrhée ou inversement d'un excès de sécheresse peut être alors observée.

On sait protéger les cheveux et la peau des effets de la lumière en y appliquant des filtres UV hydrosolubles ou non, polymériques ou non, des nanoparticules, des antioxydants, des agents complexants de métaux ou chélatants, ou des agents anti-radicaux libres.

Pour mieux démêler et empêcher une certaine rêcheur des cheveux, on peut aussi leur appliquer des agents de conditionnement monomériques ou polymériques, hydrosolubles ou non.

Les compositions de l'art antérieur permettent de résoudre, dans une certaine mesure, les problèmes évoqués, mais leur utilisation n'est pas satisfaisante. En effet, des applications répétées de ces compositions ont souvent pour effet de communiquer un toucher désagréable aux cheveux, une perte de volume et de nervosité de la chevelure, et parfois un manque de brillance.

D'autre part, on obtient avec de telles compositions un toucher gras persistant sur la peau.

Dans le domaine des compositions cosmétiques dites "rincées", tels que les shampooings, les gels douches, les démaquillants visage ou les mousses à raser, on connaît des compositions détergentes comprenant des agents bénéfiques pour la chevelure ou la peau. Ces ingrédients bénéfiques sont généralement introduits dans la composition pour faciliter le démêlage de la chevelure, améliorer les qualités du toucher de la fibre kératinique ou de la peau ou encore favoriser l'hydratation de cette dernière. Les polymères cationiques, les silicones et les émollients comme les polyols constituent dans ce cadre les ingrédients les plus couramment utilisés.

Pour obtenir l'effet bénéfique escompté, il est nécessaire d'introduire une quantité d'agent bénéfique dans la composition rincée bien supérieure à la quantité nécessaire à l'obtention de l'effet. Pour les compositions détergentes rincées, la majeure partie de l'agent bénéfique est en effet éliminée lors du rinçage de la composition, la part efficace de l'agent contenu dans la composition est de ce fait très faible.

Cependant, à la date d'aujourd'hui il n'existe pas de compositions détergentes rincées permettant d'accroître de façon majeure la quantité d'agent bénéfique vectorisé après rinçage. De telles compositions permettraient d'utiliser beaucoup moins d'agent bénéfique pour apporter le même niveau d'effet bénéfique.

La demanderesse a découvert de manière surprenante qu'en utilisant, dans un milieu cosmétiquement acceptable, au moins une association de quatre tensioactifs et d'un polymère cationique particulier en combinaison avec au moins un agent bénéfique pour les matières kératiniques, on pouvait remédier aux inconvénients cités ci-dessus.

En particulier, il est possible d'augmenter le dépôt de l'agent bénéfique des matières kératiniques et par la même d'augmenter l'efficacité desdits agents bénéfiques ou de diminuer la quantité dudit agent utilisée.

L'invention a donc pour objet une composition cosmétique détergente, caractérisée par le fait qu'elle comprend, dans un milieu aqueux cosmétiquement acceptable, (A) au moins un tensioactif anionique comprenant au moins un groupe choisi parmi sulfate, sulfonate (B) au moins un tensioactif anionique carboxylique différent du tensioactif cité en (A), (C) au moins un tensioactif amphotère , (D) au moins un tensioactif non ionique alkylpolyglycoside, (E) au moins un polymère cationique ayant une densité de charge cationique supérieure à 4 meq/g et (F) au moins un agent bénéfique des matières kératiniques différent de (E), le rapport en poids tensioactif anionique sulfate ou sulfonate / tensioactif amphotère allant de 0,1 à 2.

Sans vouloir limiter la présente invention à une quelconque théorie, il semblerait exister lors du rinçage, des interactions et/ou des affinités particulières entre l'agent bénéfique des matières kératiniques, l'association de tensioactifs conformes à l'invention, le polymère cationique et les cheveux, qui favorisent un dépôt régulier, important et durable desdites agents bénéfiques des matières kératiniques et du polymère cationique à la surface desdits cheveux, ce dépôt qualitatif et quantitatif étant probablement l'une des causes de l'amélioration observée au niveau des propriétés finales, en particulier la facilité de coiffage, le démêlage, le lissage, la douceur et la brillance des cheveux traités lorsqu'on utilise un agent conditionneur.

Toutes ces découvertes sont à la base la présente invention.

Un autre objet de l'invention concerne l'utilisation d'au moins une association comprenant (A) au moins un tensioactif anionique comprenant au moins un groupe choisi parmi sulfate, sulfonate, (B) au moins un tensioactif anionique carboxylique différent du tensioactif cité en (A), (C) au moins un tensioactif amphotère, (D) au moins un tensioactif non ionique alkylpolyglycoside, (E) au moins un polymère cationique ayant une densité de charge cationique supérieure à 4 meq/g, le rapport en poids tensioactif anionique sulfate ou sulfonate / tensioactif amphotère allant de 0,1 à 2 dans, ou pour la fabrication d'une composition cosmétique comprenant un agent bénéfique des matières kératiniques.

L'invention a également pour objet l'utilisation d'au moins une association comprenant (A) au moins un tensioactif anionique comprenant au moins un groupe choisi parmi sulfate, sulfonate, (B) au moins un tensioactif anionique carboxylique différent du tensioactif cité en (A), (C) au moins un tensioactif amphotère, (D) au moins un tensioactif non ionique alkylpolyglycoside, (E) au moins un polymère cationique ayant une densité de charge cationique supérieure à 4 meq/g le rapport en poids tensioactif anionique sulfate ou sulfonate / tensioactif amphotère allant de 0,1 à 2, dans une composition comprenant un agent bénéfique des matières kératiniques pour augmenter l'efficacité de cet agent bénéfique des matières kératiniques.

La présente invention a aussi pour objet l'utilisation d'au moins une association comprenant (A) au moins un tensioactif anionique comprenant au moins un groupe choisi parmi sulfate, sulfonate, (B) au moins un tensioactif anionique carboxylique différent du tensioactif cité en (A), (C) au moins un tensioactif amphotère, (D) au moins un tensioactif non ionique alkylpolyglycoside, (E) au moins un polymère cationique ayant une densité de charge cationique supérieure à 4 meq/g, le rapport en poids tensioactif anionique sulfate ou sulfonate / tensioactif amphotère allant de 0,1 à 2 dans une composition comprenant un agent bénéfique des matières kératiniques pour améliorer le dépôt et/ou la fixation dudit agent bénéfique sur les matières kératiniques.

L'invention a également pour objet l'utilisation en cosmétique des compositions ci-dessus pour le nettoyage et /ou le démaquillage et/ou le conditionnement des matières kératiniques telles que les cheveux et la peau.

Les différents objets de l'invention vont maintenant être détaillés. L'ensemble des significations et définitions des composés utilisés dans la présente invention données ci-dessous sont valables pour l'ensemble des objets de l'invention.

Selon l'invention le terme « au moins un » signifie « un », « deux », trois », .... ou plusieurs.

On appelle agent bénéfique pour les matières kératiniques, un agent susceptible notamment de protéger, d'embellir, de conditionner, de traiter et/ou de faciliter la mise en forme les matières kératiniques, et en particulier les cheveux.

### (A) Tensioactifs sulfate(s) ou sulfonate(s)

Selon l'invention, les tensioactifs anioniques sulfate(s) ou sulfonate(s) sont des tensioactifs anioniques comportant au moins une fonction sulfate (-OSO₃H ou - OSO₃⁻) et/ou une fonction sulfonate (-SO₃H ou -SO₃⁻) et ne comprenant pas de fonction cationique.

Les tensioactifs anioniques sulfates ou sulfonates utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en C₈-C₁₄ et plus particulièrement ceux en C₁₂-C₁₄. Ces sels comprennent de préférence de 2 à 5 groupements d'oxyde d'éthylène.

On utilise de préférence parmi ceux-ci les alkyl(C₁₂-C₁₄) sulfates de sodium, de triéthanolamine, de magnésium ou d'ammonium, les alkyl (C₁₂-C₁₄)éthersulfates de sodium, d'ammonium ou de magnésium oxyéthylénés à 2,2 moles d'oxyde d'éthylène. On peut aussi utiliser à titre de tensioactif sulfate ou sulfonate le cocoyl iséthionate de sodium et les méthyl acyl taurate.

Les tensioactifs anioniques sulfates ou sulfonates sont généralement présents à raison de 1 % à 50 % en poids, de préférence de 2 à 25 % en poids, particulièrement de 2 à 25% en poids, plus particulièrement de 3 à 20%, mieux de 3 à 10% en poids et en core mieux de 3,5 à 8 % en poids par rapport au poids total de la composition.

### (B) Tensioactifs anioniques carboxyliques

Selon l'invention, les tensioactifs anioniques carboxyliques sont des tensioactifs anioniques comportant au moins une fonction carboxylique (-COOH) éventuellement sous forme de sel (-COO⁻).

Les tensioactifs anioniques de type carboxyliques différents des tensioactifs (A) ne comprennent de préférence pas de fonction sulfate ou sulfonate et peuvent être notamment choisis parmi les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés et leurs sels, les acides alkyl(C₆-C₂₄) amido éther carboxyliques polyoxyalkylénés et leurs sels, en particulier ceux comportant de 2 à 50 groupements oxyde d'alkylène en particulier d'éthylène tels que les composés proposés par la société KAO sous les dénominations AKYPO, les acyl(C₆-C₂₄) sarcosinates et leurs sels, les acyl(C₆-C₂₄)lactylates et leurs sels et les acyl(C₆-C₂₄) glutamates. On peut également utiliser les esters d'alkyl(C₆-C₂₄)polyglycosides carboxyliques tels que les alkylglucoside acétates, les alkylglucoside citrates et les alkylpolyglycoside tartrate. De tels produits sont notamment vendus sous les dénominations de EUCAROL APG/EC et EUCAROL APG/ET par la société LAMBERTI et PLANTAPON LGC SORB par la société COGNIS.

On peut également utiliser les mélanges de ces tensioactifs.

Les sels sont en particulier choisis parmi les sels alcalins, notamment de sodium, les sels d'ammonium, les sels d'amines, les sels d'aminoalcools tels la triéthanolamine ou la monoéthanolamine et les sels de magnésium.

On utilise de préférence les acides alkyl (C₆-C₂₄) éther carboxyliques polyoxyalkylénés et leurs sels, les acides alkyl(C₆-C₂₄) amido éther carboxyliques polyoxyalkylénés et leurs sels en particulier ceux comportant de 2 à 15 groupements oxyde d'alkylène, les esters d'alkyl(C₆-C₂₄)polyglycosides carboxyliques et leurs sels et les acyl(C6-C24)glutamates et leurs sels et leurs mélanges.

On utilise plus particulièrement les acides alkyl (C₆-C₂₄) éther carboxyliques polyoxyalkylénés et leurs sels, les acides alkyl(C₆-C₂₄) amido éther carboxyliques polyoxyalkylénés en particulier ceux comportant de 2 à 15 groupements oxyde d'alkylène, et leurs sels et leurs mélanges.

Les tensioactifs anioniques du type acides ou sels d'éthers carboxyliques polyoxyalkylénés sont en particulier ceux qui répondent à la formule (1) suivante : 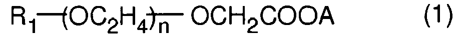 dans laquelle :
R1 représente un radical ou un mélange de radicaux alkyle ou alcényle linéaire ou ramifié en C₈-C₂₂, un radical alkyl(C₈-C₉)phényle, un radical R2CONH-CH₂-CH₂- avec R2 désignant un radical alkyle ou alcényle linéaire ou ramifié en C₁₁-C₂₁,
et n est un nombre entier ou décimal (valeur moyenne) pouvant varier de 2 à 24 et de préférence de 2 à 10, le radical alkyle ayant entre 6 et 20 atomes de carbone environ, de préférence de 8 à 18 atomes de carbone et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Les acides éther carboxylique oxyalkylénés ou leurs sels utilisés de préférence selon la présente invention sont choisis parmi ceux de formule (I) dans laquelle R1 désigne un radical ou un mélange de radicaux alkyl(C₁₂-C₁₄), cocoyle, oléyle; un radical nonylphényle ou octylphényle, A désigne un atome d'hydrogène ou de sodium, et n varie de 2 à 20 et de préférence 2 à 10.

Plus préférentiellement encore, on utilise des composés de formule (I) dans laquelle R désigne un radical alkyl(C₁₂), A désigne un atome d'hydrogène ou de sodium, et n varie de 2 à 10.

Parmi les produits commerciaux, on peut utiliser de préférence les produits vendus par la société CHEM Y sous les dénominations :
AKYPO® NP 70 (R=nonylphényle, n=7, p=0, A=H)
AKYPO® NP 40 (R=nonylphényle, n=4, p=0, A=H)
AKYPO® OP 40 (R=octylphényle, n=4, p=0, A=H)
AKYPO® OP 80 (R=octylphényle, n=8, p=0, A=H)
AKYPO® OP 190 (R=octylphényle, n=19, p=0, A=H)
AKYPO® RLM 38 (R= alkyl(C₁₂-C₁₄), n=3,8, p=0, A=H)
AKYPO® RLM 38 NV (R= alkyl(C₁₂-C₁₄), n=4, p=0, A=Na)
AKYPO® RLM 45 (R= alkyl(C₁₂-C₁₄), =4,5, p=0, A=H)
AKYPO® RLM 45 NV (R= alkyl(C₁₂-C₁₄), n=4,5, p=0, A=Na)
AKYPO® RLM 100 (R= alkyl(C₁₂-C₁₄), n=10, p=0, A=H)
AKYPO® RLM 100 NV (R= alkyl(C₁₂-C₁₄), n=10, p=0, A=Na)
AKYPO® RLM 130 (R= alkyl(C₁₂-C₁₄), n=13, p=0, A=H)
AKYPO® RLM 160 NV (R= alkyl(C₁₂-C₁₄), n=16, p=0, A=Na)
ou par la société SANDOZ sous les dénominations :
SANDOPAN DTC-Acid (R= alkyl(C₁₃), n=6, p=0, A=H)
SANDOPAN DTC (R= alkyl(C₁₃), n=6, p=0, A=Na)
SANDOPAN LS 24 (R= alkyl(C₁₂-C₁₄), n=12, p=0, A=Na)
SANDOPAN JA 36 (R= alkyl(C₁₃), n=18, p=0, A=H),
et plus particulièrement, les produits vendus sous les dénominations suivantes :
AKYPO® RLM 45
AKYPO® RLM 100
AKYPO® RLM 38.

Les tensioactifs anioniques carboxyliques différents des tensioactifs cités en A) sont généralement présents à raison de 0,5 % à 15 % en poids, de préférence de 1 à 10 % en poids, plus particulièrement de 1,5 à 8% en poids et encore plus préférentiellement de 2 à 5% en poids par rapport au poids total de la composition.

### (C) Tensioactif(s) amphotère(s) :

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits décrits dans les brevets US-2 528 378 et US-2 781 354 et de structures :

R₂ -CONHCH₂CH₂ -N(R₃)(R₄)(CH₂COO-) (2)

dans laquelle : R₂ CO désigne un radical acyle en C6-C24, par exemple un radical présent dans l'huile de coprah hydrolysée, un radical octoyle, décoyle ou dodécanoyle et leurs mélanges, R₃ désigne un groupement bêta-hydroxyéthyle et R₄ un groupement carboxyméthyle;
et

R₂,-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R'₂ CO désigne un radical acyle en C6-C24, par exemple un radical présent dans l'huile de coprah hydrolysée ou l'huile de lin, un radical octoyle, décoyle ou dodécanoyle, stéaroyle ou isostéaroyle, oléoyle et leurs mélanges.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le disodium cocoamphodiacetate commercialisé sous la dénomination commerciale MIRANOL^{®} C2M concentré par la société RHODIA CHIMIE.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines, en particulier la cocamidopropylbétaine telles que la TEGOBETAINE^{®} F50 commercialisée par la société GOLDSCHMIDT.

Le ou les tensio-actif(s) amphotère(s) sont généralement présents dans des quantités allant de 0,1 à 20 % en poids, de préférence de 1 à 15 % en poids, et plus particulièrement de 2 à 10% en poids et mieux de 3 à 7 % en poids par rapport au poids total de la composition.

### (D) Tensioactif(s) non ionique(s) alkylpolyglycoside:

Les tensioactifs non ioniques alkypolyglycosides, peuvent être plus particulièrement représenté par la formule générale suivante :

R₁O-(R₂O)ₜ (G)ᵥ (4)

dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (4) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (4), peut aller de 1 à 15, de préférence de 1 à 4. Le degré moyen de polymérisation est plus particulièrement compris entre 1 et 2 et encore plus préférentiellement de 1,1 à 1,5.

Les liaisons glycosidiques entre les motifs sucre sont de type 1-6 ou 1-4 et de préférence 1-4.

Des composés de formule (4) sont notamment représentés par les produits vendus par la société COGNIS sous les dénominations PLANTAREN^{®} (600 CS/U, 1200 et 2000) ou PLANTACARE^{®} (818, 1200 et 2000). On peut également utiliser les produits vendus par la société SEPPIC sous les dénominations TRITON CG 110 (ou ORAMIX CG 110) et TRITON CG 312 (ou ORAMIX^{®} NS 10), les produits vendus par la société B.A.S.F. sous la dénomination LUTENSOL GD 70 ou encore ceux vendus par la société CHEM Y sous la dénomination AG10 LK.

On peut également utiliser par exemple, l'Alkyl en C8/C16 polyglucoside 1,4 en solution aqueuse à 53% commercialisé par COGNIS sous la référence PLANTACARE^{®} 818 UP.

Le ou les tensio-actif(s) non ionique(s) de type alkylpolyglycoside sont généralement présents dans des quantités allant de 0,1 à 20 % en poids, de préférence de 1 à 15 % en poids, et plus particulièrement de 2 à 10% en poids et mieux de 3 à 8 % en poids par rapport au poids total de la composition.

La quantité minimale de tensioactifs est celle suffisante pour conférer à la composition finale un pouvoir moussant et/ou détergent satisfaisant, et des quantités trop importantes de tensioactifs n'apportent pas vraiment d'avantages supplémentaires.

Ainsi, selon l'invention, la quantité totale de tensioactifs peut représenter de 4 % à 50 % en poids, de préférence de 6 % à 35 % en poids, et encore plus préférentiellement de 8 % à 25 % en poids, du poids total de la composition finale.

Le rapport en poids tensioactif anionique sulfate ou sulfonate / tensioactif amphotère va de préférence de 0,1 à 2, plus particulièrement de 0,5 à 1 et encore plus préférentiellement de 0,7 à 0,8.

Le rapport en poids tensioactif anionique sulfate ou sulfonate / tensioactif anionique carboxylique va de préférence de 0,1 à 10 et encore plus préférentiellement de 0,5 à 5 et mieux de 1 à 3.

Le rapport en poids tensioactif anionique carboxylique/ tensioactif amphotère va de préférence de 0,1 à 10 et encore plus préférentiellement de 0,2 à 5 et mieux de 0,3 à 2.

La composition cosmétique selon l'invention comprend un ou plusieurs polymères cationiques dont la densité de charge cationique est supérieure à 4 milliéquivalents par gramme (meq/g), de préférence supérieure ou égale à 5 milliéquivalents par gramme (meq/g), de préférence allant de 5 à 20 méq/g et plus particulièrement de 5,5 à 10 meq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl, généralement à un pH d'environ 7 à température ambiante.

Les polymères cationiques ayant une densité de charge cationique supérieure à 4 méq/g, utilisables conformément à la présente invention, peuvent être choisis parmi tous ceux déjà connus en soi comme améliorant les propriétés cosmétiques des cheveux traités par des compositions, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière générale, au sens de la présente invention, l'expression "polymère cationique" désigne tout polymère comprenant des groupes cationiques et/ou des groupes ionisables en groupes cationiques.

Les polymères cationiques sont choisis parmi ceux qui comprennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Parmi ces polymères, on peut citer :
(1) les homopolymères ou copolymères dérivés d'esters ou d'amides acryliques ou méthacryliques et comportant au moins un des motifs de formules suivantes: 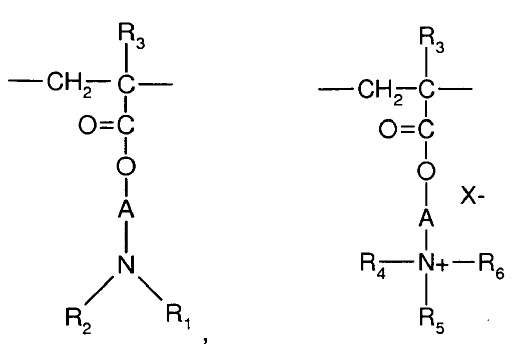 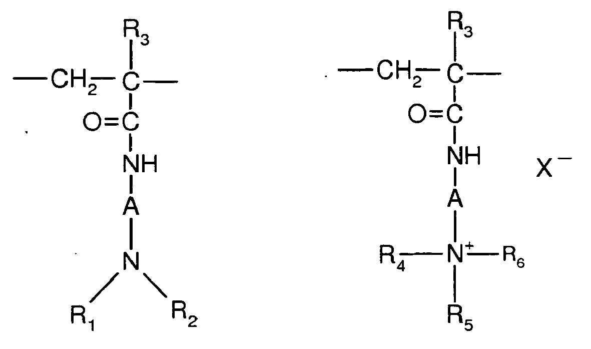 dans lesquelles:
   R₃ , identiques ou différents, désignent un atome d'hydrogène ou un radical CH₃; A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle
   - les copolymères d'acrylamide et de chlorure de méthacryloyloxyéthyltriméthylammonium décrit par exemple dans la demande de brevet EP-A-080976
   - le copolymères d'acrylamide et de méthosulfate de méthacryloyloxyéthyltriméthylammonium,
   - les copolymères vinylpyrrolidone / acrylate ou méthacrylate de dialkylaminoalkyle quaternisés ou non. Ces polymères sont décrits en détail dans les brevets français 2.077.143 et 2.393.573,
   - les terpolymères méthacrylate de diméthyl amino éthyle/ vinylcaprolactame/ vinylpyrrolidone,
   - les copolymères vinylpyrrolidone / méthacrylamidopropyl dimethylamine.
   - et les copolymères vinylpyrrolidone / méthacrylamide de diméthylaminopropyle quaternisé.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle.
   Parmi ces dérivés, on peut citer plus particulièrement les polymères acide adipique/diméthylaminohydroxypropyl/diéthylène triamine
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone.
(7) les cyclopolymères d'alkyl diallyl amine ou de dialkyl diallyl ammonium tels que les homopolymères ou copolymères comportant comme constituant principal de la chaîne des motifs répondant aux formules (5I) ou (5') : 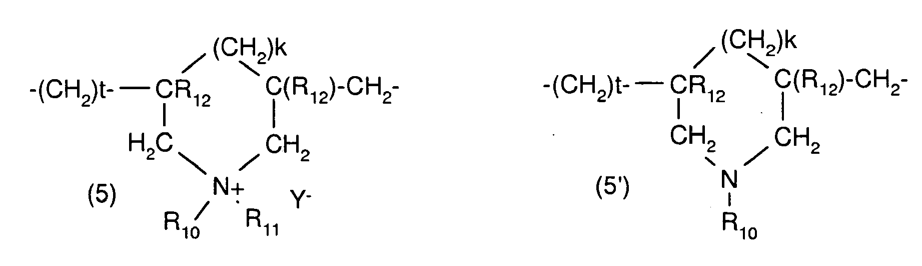 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement les homopolymères de chlorure de diméthyldiallylammonium vendus notamment sous la dénomination "MERQUAT 100" par la société NALCO (et ses homologues de faibles masses molaires moyenne en poids) et les copolymères de chlorure de diallyldiméthylammonium et d'acrylamide
(8) les polymères de diammonium quaternaire notamment ceux contenant des motifs récurrents répondant à la formule : 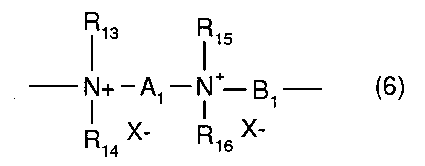 formule (6) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
      a) un reste de glycol de formule : -O-Z-O-, où Z désigne un radical hydrocarboné linéaire ou ramifié ou un groupement répondant à l'une des formules suivantes :

         -(CH2-CH2-O)x-CH2-CH2-

         -[CH2-CH(CH3)-O]y-CH2-CH(CH3)-

         où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;
   De préférence, X⁻ est un anion tel que le chlorure ou le bromure.
   Ces polymères ont une masse molaire moyenne en nombre généralement comprise entre 1000 et 100000.
   On peut utiliser plus particulièrement les polymères qui sont constitués de motifs récurrents répondant à la formule : 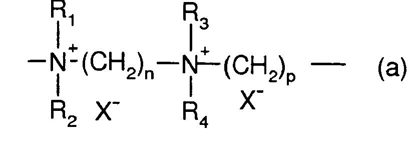 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires comprenant des motifs de formule (7): 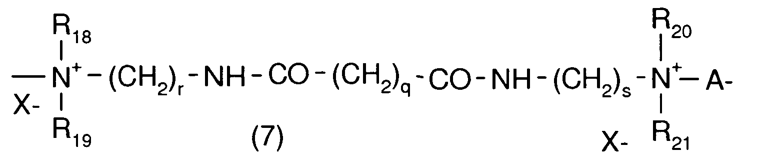 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β -hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1 ", "Mirapol® AZ1 " et "Mirapol® 175" vendus par la société Miranol.
(10) Les copolymères quaternaires de vinyllactame (vinylpyrrolidone et/ou vinylcaprolactame) et de vinylimidazole
(11) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi ces polymères cationiques, on préfère utiliser ceux des familles (1), (7), (8), (9) et (10).

Parmi tous les polymères cationiques susceptibles d'être utilisés dans le cadre de la présente invention, on préfère mettre en oeuvre les cyclopolymères cationiques, en particulier les homopolymères de chlorure de diméthyldiallylammonium, vendus sous les dénominations « MERQUAT 100 », » par la société NALCO, les polymères de diammonium quaternaire, les polyéthylèneimines et leurs mélanges.

Le ou les polymères cationiques ayant une densité cationique supérieure à 4maeq/g sont généralement présents dans des quantités allant de 0,01 à 20 % en poids, de préférence de 0,05 à 10 % en poids, et plus particulièrement de 0,1 à 5% en poids et mieux de 0,2 à 2 % en poids par rapport au poids total de la composition.

Ledit agent bénéfique pour les matières kératiniques peut être choisi parmi :
(1) les saccharides, les oligosaccharides, les polysaccha-rides hydrolysés ou non, modifiés ou non,
(2) les acides aminés, les oligopeptides, les peptides, les protéines hydrolysées ou non, modifiées ou non,
(3) les acides et alcools gras ramifiés ou non,
(4) les cires animales, végétales ou minérales,
(5) les céramides et les pseudo-céramides,
(6) les acides organiques hydroxylés,
(7) les filtres UV,
(8) les antioxydants et les agents anti-radicaux libres,
(9) les chélatants,
(10) les agents antipelliculaires,
(11) les agents régulateurs de séborrée,
(12) les agents apaisants,
(13) les tensioactifs cationiques,
(14) les silicones organomodifiées ou non,
(15) les huiles minérales, végétales ou animales,
(16) les polyisobutènes et poly(alpha-oléfines),
(17) les esters gras, de préférence ayant de 15 à 50 atomes de carbone,
(18) les polymères anioniques solubles ou dispersés,
(19) les polymères non-ioniques solubles ou dispersés,
et leurs mélanges.

La composition selon l'invention peut comprendre un ou plusieurs saccharides, oligosaccharides, polysaccharides hydrolysés ou non, modifiés ou non, identiques ou non. D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

A titre d'exemples de saccharides, oligosaccharides, polysaccha-rides hydrolysés ou non, modifiés ou non, utilisables dans l'invention, on peut notamment citer les glucanes, les amidons modifiés ou non (tels que ceux issus, par exemple, de céréales comme le blé, le maïs ou le riz, de légumes comme le pois blond, de tubercules comme les pommes de terre ou le manioc) et différents du bétaïnate d'amidon tel que décrit ci-dessus, l'amylose, l'amylopectine, le glycogène les dextranes, les β-glucanes, les celluloses et leurs dérivés (méthylcelluloses, hydroxyalkylcelluloses, éthylhydroxyéthylcelluloses, carboxyméthyl-celluloses), les fructosanes, l'inuline, la levane, les mannanes, les xylanes, les lignines, les arabanes, les galactanes, les galacturonanes, la chitine, les glucoronoxylanes, les arabinoxylanes, les xyloglucanes, les galactomannanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les gommes de guar et les gommes de xanthane, et leurs mélanges.

La composition selon l'invention peut comprendre un ou plusieurs acides aminés, oligopeptides, peptides, protéines hydrolysées ou non, modifiées ou non. Comme acides aminés, on peut citer, par exemple, la cystéine, la lysine, l'alanine, la N-phénylalanine, l'arginine, la glycine, la leucine, et leurs mélanges. A titre d'oligopeptides, de peptides, de protéines hydrolysées ou non, modifiées ou non, que l'on peut utiliser dans la compositon selon l'invention, on peut notamment citer les hydrolysats de protéines de laine ou de soie, modifiées ou non, les protéines végétales telles que les protéines de blé.

La composition selon l'invention peut comprendre un ou plusieurs acides et alcools gras ramifiés ou non. Parmi les acides gras convenant dans la présente invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges. Les alcools gras utilisables dans la présente invention, comprennent notamment les alcools en C₈-C₃₀ comme, par exemple, les alcools palmitylique, oléylique, linoléylique, myristylique, stéarylique et laurylique, et leurs mélanges.

La composition selon l'invention peut comprendre une ou plusieurs cires animales, végétales ou minérales.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines ou les ozokérites, et leurs mélanges.

La composition selon l'invention peut comprendre un ou plusieurs céramides et/ou pseudo-céramides. On peut citer en particulier les céramides des classes I, II, III et V selon la classification de DAWNING, et leurs mélanges, et plus particulièrement la N-oléyldéshydrosphingosine.

La composition selon l'invention peut comprendre un ou plusieurs acides organiques hydroxylés choisis parmi ceux bien connus et utilisés dans la technique. On peut notamment citer l'acide citrique, l'acide lactique, l'acide tartrique, l'acide malique et leurs mélanges.

La composition selon l'invention peut comprendre un ou plusieurs filtres solaires actifs dans l'UV-A et/ou l'UV-B bien connus de l'homme de métier. On peut notamment citer les dérivés du dibenzoylméthane tels que le 4-méthyldibenzoylméthane, le 4-isopropyldibenzoylméthane, le 4-tert-butyldibenzoylméthane, le 2,4-diméthyldibenzoylméthane, le 4-tert-butyl-4'-diisopropyldibenzoyl-méthane, l'acide p-amino-benzoïque et ses esters tels que le p-diméthylaminobenzoate de 2-éthylhexyle et le p-aminobenzoate d'éthyle N-propoxylé, les salicylates tels que le salicylate de triéthanolamine, les esters d'acide cinnamique tels que le 4-méthoxy-cinnamate de 2-éthylhexyle, le diisopropylcinnamate de méthyle, l'anthranilate de menthyle, les dérivés de benzotriazole, les dérivés de triazine, les dérivés de β,β'-diphénylacrylate tels que le 2-cyano-3,3-diphénylacrylate de 2-éthylhexyle et le 2-cyano-3,3-diphénylacrylate d'éthyle, l'acide 2-phénylbenzimidazole-5-sulfonique et ses sels, les dérivés de benzophénone, les dérivés du benzylidène-camphre, les filtres siliconés, etc., et leurs mélanges.

A titre d'antioxydants et d'agents anti-radicaux libres utilisables dans la présente invention, on peut citer, par exemple, l'acide ascorbique, des composés ascorbylés tels que le dipalmitate d'ascorbyle, la t-butylhydroquinone, les polyphénols tels que le phloroglucinol, le sulfite de sodium, l'acide érythorbique, les flavonoïdes et leurs mélanges.

La composition selon l'invention peut comprendre un ou plusieurs chélatants choisis notamment parmi l'EDTA (acide éthylènediaminetétraacétique) et ses sels tels que l'EDTA disodique et l'EDTA dipotassique, les composés phosphatés tels que le métaphosphate de sodium, l'hexamétaphosphate de sodium, le pyrophosphate tétrapotassique, les acides phosphoniques et leurs sels tels que les sels de l'acide éthylènediaminetétraméthylènephospho-nique, et leurs mélanges.

La composition selon l'invention peut comprendre un ou plusieurs agents antipelliculaires choisis, par exemple, parmi :
- le chlorure de benzéthonium, le chlorure de benzalkonium, la chlorhexidine, la chloramine T, la chloramine B, la 1,3-dibromo-5,5-diméthylhydantoïne, la 1,3-dichloro 5,5-diméthylhydantoïne, la 3-bromo 1-chloro 5,5-diméthylhydantoïne, le N-chlorosuccinimide ;
- les dérivés de 1-hydroxy-2-pyridone tels que, par exemple, le 1-hydroxy-4-méthyl-2-pyridone, le 1-hydroxy-6-méthyl-2-pyridone et le 1-hydroxy-4,6-diméthyl-2-pyridone ;
- les trihalogénocarbamides ;
- le triclosan ;
- les composés azolés tels que le climbazole, le kétoconazole, le clotrinazole, l'econazole, l'isoconazole et le miconazole b ;
- les polymères antifongiques tels que l'amphotéricine B ou la nystatine ;
- les sulfures de sélénium ;
- le soufre sous ses différentes formes, le sulfure de cadmium, l'allantoïne, les goudrons de houille ou de bois et leurs dérivés en particulier, l'huile de cade, l'acide undécylénique, l'acide fumarique, les allylamines telles que la terbinafine ;
- ou un mélange de ces agents antipelliculaires.

On peut également les utiliser sous forme de leurs sels d'addition d'acides physiologiquement acceptables, notamment sous forme de sels d'acides sulfurique, nitrique, thiocyanique, chlorhydrique, bromhydrique, iodhydrique, phosphorique, acétique, benzoïque, glycolique, acéturique, succinique, nicotinique, tartrique, maléique, palmitique, méthane sulfonique, propanoïque, 2-oxopropanoïque, propanedioïque, 2-hydroxy-1,4-butanedioïque, 3-phényl-2-propènoïque, -hydroxybenzèneacétique, éthanesulfonique, 2-hydroxyéthanesulfonique, 4-méthylbenzènesulfonique, 4-amino-2-hydroxybenzoïque, 2-phénoxybenzoïque, 2-acétyloxybenzoïque, picrique, lactique, citrique, malique, oxalique et d'aminoacides.

Les agents antipelliculaires mentionnés ci-dessus peuvent aussi le cas échéant être utilisés sous forme de leurs sels d'addition de bases organiques ou inorganiques physiologiquement acceptables. Des exemples de bases organiques sont notamment les alcanolamines de faibles poids moléculaires telles que l'éthanolamine, la diéthanolamine, la N-éthyléthanolamine, la triéthanolamine, le diéthylaminoéthanol, le 2-amino-2-méthylpropanedione; les bases non-volatiles telles que l'éthylènediamine, l'hexaméthylènediamine, la cyclohexylamine, la benzylamine, la N-méthylpipérazine; les hydroxydes d'ammonium quaternaires, par exemple, le triméthylbenzyl hydroxyde ; la guanidine et ses dérivés, et particulièrement ses dérivés alkylés. Des exemples de bases inorganiques sont notamment les sels de métaux alcalins, comme le sodium ou potassium; les sels d'ammonium, les sels de métaux alcalino-terreux, comme le magnésium, calcium ; les sels de métaux di, tri ou tétravalents cationiques, comme le zinc, l'aluminium et le zirconium. Les alcanolamines, l'éthylènediamine et les bases inorganiques telles que les sels de métaux alcalins sont préférées.

La composition selon l'invention peut comprendre un ou plusieurs agents régulateurs de séborrhée tels que le succinylchitosane et la poly-beta-alanine, et leurs mélanges

La composition selon l'invention peut comprendre un ou plusieurs agents apaisants tels que l'azulène et l'acide glycyrrhétinique, et leurs mélanges.

La composition selon l'invention peut comprendre un ou plusieurs tensioactifs cationiques bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyl-trialkylammo-nium, de trialkylbenzylammonium, de trialkylhydroxy-alkylammonium ou d'alkylpyridinium ; les dérivés d'imidazoline ; ou les oxydes d'amines à caractère cationique.

Les silicones utilisables conformément à l'invention peuvent être solubles ou insolubles dans la composition, et elles peuvent être en particulier des polyorganosiloxanes insolubles dans la composition de l'invention ; elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique :
   avec D : 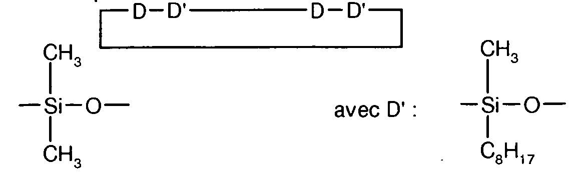 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".
   Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.
   Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.
   Parmi les silicones organomodifiées, on peut citer les polyorganosiloxanes comportant :
   - des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
   - des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
   - des groupements thiols, comme les produits commercialisés sous les dénominations "GP 72 A" et "GP 71" de GENESEE ;
   - des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
   - des groupements hydroxylés, comme les polyorganosiloxanes à fonction hydroxyalkyle décrits dans la demande de brevet français FR-A-85 16334 .
   - des groupements acyloxyalkyle tels que, par exemple, les polyorganosiloxanes décrits dans le brevet US-A-4957732.
   - des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
   - des groupements hydroxyacylamino, comme les polyorgano-siloxanes décrits dans la demande EP 342 834. On peut citer, par exemple, le produit Q2-8413 de la société DOW CORNING.
   La composition selon l'invention peut comprendre une ou plusieurs huiles minérales, végétales ou animales. On peut notamment citer comme huiles d'origine végétale, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; comme huile d'origine animale, le perhydrosqualène ; comme huiles d'origine minérale, l'huile de paraffine et l'huile de vaseline ; et leurs mélanges.
   La composition selon l'invention peut comprendre un ou plusieurs polyisobutènes et poly(alpha-oléfines), choisis parmi ceux bien connus dans la technique.
   La composition selon l'invention peut comprendre un ou plusieurs esters gras. Comme exemples, on peut notamment citer les esters d'acides gras ayant de préférence de 8 à 22 atomes de carbone comme le myristate d'isopropyle, le palmitate d'iso-propyle, le palmitate de 2-éthylhexyle, l'huile de Purcellin (octanoate de stéaryle), l'isononanoate d'isononyle ou d'isostéaryle, le lanolate d'isopropyle, et leurs mélanges. On peut également notamment citer les esters d'alcools gras ayant de préférence de 8 à 22 atomes de carbone.

La composition selon l'invention peut comprendre un ou plusieurs polymères anioniques solubles ou dispersés, bien connus en soi. Les polymères anioniques généralement utilisés dans la présente invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 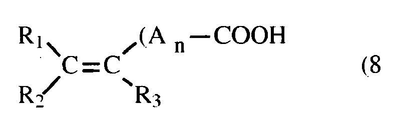 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂-COOH, phényle ou benzyle. Dans la formule (8) ci-dessus, un groupement alkyle inférieur comporte de préférence de 1 à 4 atomes de carbone et désigne en particulier, les groupements méthyle et éthyle.

Les polymères anioniques à groupements carboxyliques préférés selon l'invention sont :
A) les homo- ou copolymères d'acide acrylique ou méthacrylique ou leurs sels, et en particulier les produits vendus sous les dénominations VERSICOL^{®} E ou K par la société ALLIED COLLOID, ULTRAHOLD^{®} par la société BASF Les copolymères d'acide acrylique et d'acrylamide vendus sous la forme de leur sel de sodium sous les dénominations RETEN^{®} 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER^{®} par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER^{®} MAEX par la société BASF.
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique alpha ou beta-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ^{®} AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une -oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène-sulfonique, les sels de sodium, ayant une masse moléculaire d'environ 500 000 et d'environ 100 000 vendus respectivement sous les dénominations Flexan^{®} 500 et Flexan^{®} 130 par National Starch. Ces composés sont décrits dans le brevet FR 2198719 ;
- les sels d'acides polyacrylamide-sulfoniques tels que ceux mentionnés dans le brevet US 4 128 631, et plus particulièrement l'acide polyacrylamidoéthylpropanesulfonique vendu sous la dénomination COSMEDIA POLYMER^{®} HSP 1180 par Henkel.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG^{®} par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vynyliques, des halogénures vinyliques, des dérivés phénylvinyliques, l'acide acrylique et ses esters tels que le copolymère méthylvinyléther/anhydride maléïque monoestérifié vendu sous la dénomination GANTREZ^{®} ES 425 par la société ISP, les copolymères d'acide méthacrylique et de méthacrylate de méthyle vendus sous la dénomination EUDRAGIT^{®} L par la société ROHM PHARMA, le copolymère d'acide méthacrylique et d'acrylate d'éthyle vendu sous la dénomination LUVIMER^{®} MAEX par la société BASF, le copolymère acétate de vinyle/acide crotonique vendu sous la dénomination LUVISET^{®} CA 66 par la société BASF et le terpolymère acétate de vinyle/acide crotonique/polyéthylèneglycol sous la dénomination ARISTOFLEX^{®} A par la société BASF.

Les polymères anioniques les plus particulièrement préférés sont choisis parmi le copolymère méthylvinyléther/anhydride maléïque monoestérifié vendu sous la dénomination GANTREZ^{®} ES 425 par la société ISP, les copolymères d'acide méthacrylique et de méthacrylate de méthyle vendus sous la dénomination EUDRAGIT^{®} L par la société ROHM PHARMA, le copolymère d'acide méthacrylique et d'acrylate d'éthyle vendu sous la dénomination LUVIMER^{®} MAEX par la société BASF, le terpolymère de vinylpyrrolidone/acide acrylique/méthacrylate de lauryle vendu sous la dénomination ACRYLIDONE^{®} LM par la société ISP.

Selon l'invention, on peut également utiliser les polymères anioniques sous forme de latex ou de pseudolatex, c'est-à-dire sous forme d'une dispersion aqueuse de particules de polymères insolubles.

La composition selon l'invention peut comprendre un ou plusieurs polymères non-ioniques solubles ou dispersés. A titre de polymères non ioniques utilisables selon la présente invention, on peut notamment citer :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX^{®} 50 000, PEOX^{®} 200 000 et PEOX^{®} 500 000 ;
- les homopolymères d'acétate de vinyle tels que le produit proposé sous le nom de APPRETAN^{®} EM par la société HOECHST ou le produit proposé sous le nom de RHODOPAS^{®} A 012 par la société RHONE POULENC ;
- les copolymères d'acétate de vinyle et d'ester acrylique tels que le produit proposé sous le nom de RHODOPAS^{®} AD 310 de RHONE POULENC ;
- les copolymères d'acétate de vinyle et d'éthylène comme le produit proposé sous le nom de APPRETAN^{®} TV par la société HOECHST;
- les copolymères d'acétate de vinyle et d'ester maléique, par exemple de maléate de dibutyle tels que le produit proposé sous le nom de APPRETAN^{®} MB EXTRA par la société HOECHST ;
- les copolymères de polyéthylène et d'anhydride maléique ;
- les homopolymères d'acrylates d'alkyle et les homopolymères de méthacrylates d'alkyle tels que le produit proposé sous la dénomination MICROPEARL^{®} RQ 750 par la société MATSUMOTO ou le produit proposé sous la dénomination LUHYDRAN^{®} A 848 S par la société BASF ;
- les copolymères d'esters acryliques tels que, par exemple, les copolymères d'acrylates d'alkyle et de méthacrylates d'alkyle comme les produits proposés par la société ROHM & HAAS sous les dénominations PRIMAL^{®} AC-261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAN^{®} N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL^{®} LX 531 8 par la société NIPPON ZEON ou ceux proposés sous la dénomination CJ 0601 8 par la société ROHM & HAAS ;
- les polyuréthannes tels que les produits proposés sous les dénominations ACRYSOL^{®} RM 1020 ou ACRYSOL^{®} RM 2020 par la société ROHM & HAAS, les produits URAFLEX^{®} XP 401 UZ, URAFLEX^{®} XP 402 UZ par la société DSM RESINS ;
- les copolymères d'acétate d'alkyle et d'uréthanne tels que le produit 8538-33 par la société NATIONAL STARCH ;
- les polyamides tels que le produit ESTAPOR^{®} LO 11 proposé par la société RHONE POULENC ;
- les gommes de guar non ioniques chimiquement modifiées ou non modifiées. Les gommes de guar non ioniques non modifiées sont, par exemple, les produits vendus sous la dénomination VIDOGUM^{®} GH 175 par la société UNIPECTINE et sous la dénomination JAGUAR^{®} C par la société MEYHALL.

Les gommes de guar non-ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont, par exemple, vendues sous les dénominations commerciales JAGUAR^{®} HP8, JAGUAR^{®} HP60 et JAGUAR^{®} HP120, JAGUAR^{®} DC 293 et JAGUAR^{®} HP 105 par la société MEYHALL, ou sous la dénomination GALACTASOL^{®} 4H4FD2 par la société AQUALON.

Les groupes alkyle des polymères non ioniques comportent de préférence de 1 à 6 atomes de carbone.

Le ou les agents bénéfiques peuvent être utilisées dans les compositions conformes à l'invention dans des concentrations allant généralement de 0,001 % à 20 % en poids, de préférence de 0,005 % à 15 % en poids, et encore plus préférentiellement de 0,01 % à 12 % en poids, mieux de 0,1 à 5% en poids du poids total de la composition finale.

Les compositions détergentes selon l'invention présentent un pH final généralement compris entre 3 et 8. De préférence, ce pH est compris entre 4 et 7,5. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable. tel qu'un alcool inférieur en C₁-C₄, comme l'éthanol, l'isopropanol, le tertiobutanol, le n-butanol ; les alkylèneglycols comme le propylèneglycol, l'hexylèneglycol ou le glycérol.

La composition selon l'invention comprend de préférence au moins 30% en poids d'eau et plus particulièrement de 50 à 90% en poids et encore préférentiellement de 70 à 85% en poids par rapport au poids total de la composition.

Les compositions conformes à l'invention peuvent contenir en plus de l'association définie ci-dessus des agents régulateurs de viscosité tels que des agents épaississants. On peut citer en particulier les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-Cₗ₈ ou des alcanolamides gras en C₁₀-C₁₈ dérivés de mono ou de diéthanolamine.

Les compositions selon l'invention peuvent également contenir des additifs tels que des épaississants polymériques naturels ou synthétiques, anioniques, amphotères, zwitteroniques, non ioniques ou cationiques, associatifs ou non, des épaississants non polymériques comme des acides ou des électrolytes, des nacrants, des opacifiants, des solvants organiques, des parfums, des colorants, des particules minérales ou organiques, des conservateurs, des agents de stabilisation du pH.

L'homme de métier veillera à choisir les éventuels additifs et leur quantité de manière à ce qu'ils ne nuisent pas aux propriétés des compositions de traitement.

Ces additifs sont présents dans la composition selon l'invention en une quantité allant de 0 à 20 % en poids par rapport au poids total de la composition.

Bien entendu, l'homme du métier veillera à choisir ce ou ces éventuels composés complémentaires et/ou leurs quantités les propriétés cosmétiques attachées intrinsèquement à la composition conforme à l'invention ne soient pas, ou substantiellement pas, altérées par la ou les adjonctions envisagées.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels, de mousses, de cires, d'émulsions E/H, H/E ou d'émulsions multiples et elles conviennent principalement au lavage, au soin des matières kératiniques en particulier des cheveux et de la peau et encore plus particulièrement des cheveux.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage avec de l'eau, l'opération pouvant être répétée une ou plusieurs fois.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés. Les pourcentages sont en matières actives.

### EXEMPLES 1 à 4

On a réalisé les compositions de shampooing conformes à l'invention suivantes :

| *En gMA* | **1** | **2** | **3** | **4** |
|---|---|---|---|---|
| Coco glucoside [1] | 5 | 5 | 5 | 5 |
| Coco [2] amidopropyl bétaine | 5,4 | 5,4 | 5,4 | 5,4 |
| Lauryl ether (5 OE) carboxylate de sodium [3] | 3 | 3 | 3 | 3 |
| Sodium lauryl ether sulfate de sodium [4] | 4 | 4 | 4 | 4 |
| Polyquaternium-6 [5] | 0,4 | 0,4 | 0,4 | 0,4 |
| Polydiméthylsiloxane (Dimethicone) [6] | 2,6 | 1 | 0,5 | 0,1 |
| Conservateur | qs | qs | qs | qs |
| Parfum | qs | qs | qs | qs |
| Acide citrique | qs pH 6,5 | qs pH 6,5 | qs pH 6,5 | qs pH 6,5 |
| Eau qsp | 100 g | 100 g | 100 g | 100 g |

| | | | | |
|---|---|---|---|---|
| [1] PLANTACARE 818 UP commercialisé par COGNIS [2] DEHYTON AB 30 commercialisé par COGNIS [3] AKYPO RLM 45 CA commercialisé par KAO [4] TEXAPON N 702 commercialisé par COGNIS [5] MERQUAT 100 commercialisé par NALCO [6] DC 200 FLUID 60000 CS commercialisé par DOW CORNING | | | | |

1 gramme de chaque formule est appliqué sur une mèche de cheveux naturels bruns caucasiens de 25cm de longueur environ, pesant 2,7g.

Les mèches sont malaxées 15 secondes, laissées posées 5 minutes puis rincées 10 secondes (eau 30°C / débit 301/h) et enfin peignées. Les mèches humides sont ensuite séchées au casque 30 minutes à 70°C.

Les précédentes opérations sont renouvelées 2 fois.

Les shampooings réalisés selon l'invention montrent de très bonnes qualités d'usage (flash foaming, rinçabilité, qualité du toucher de la fibre après rinçage).

On effectue un dosage de la silicone sur les mèches séchées.

Le dosage de la quantité de silicone déposée s'effectue par spectroscopie IRTF directement sur les mèches de cheveux à l'aide d'un cristal ATR Zn-Se. La quantité de silicone déposée est définie par l'absorbance mesurée sur une des bandes caractéristiques d'absorption des silicones. La bande située à 1262 cm⁻¹ est choisie pour réaliser la mesure.

L'intensité d'absorbance mesurée sur cette bande caractéristique est ramenée à un 1 pour le shampooing témoin. Cette unité constitue l'unité arbitraire de mesure (U.A.).

Le shampooing témoin est constitué par une composition commerciale contenant une association de tensioactif anionique et amphotère, un polymère cationique et la même dimethicone à une concentration de 2,6 % identique à celle de la composition de l'exemple 1. Le shampooing témoin est appliqué dans les mêmes conditions que les compositions selon l'invention.

### Résultats :

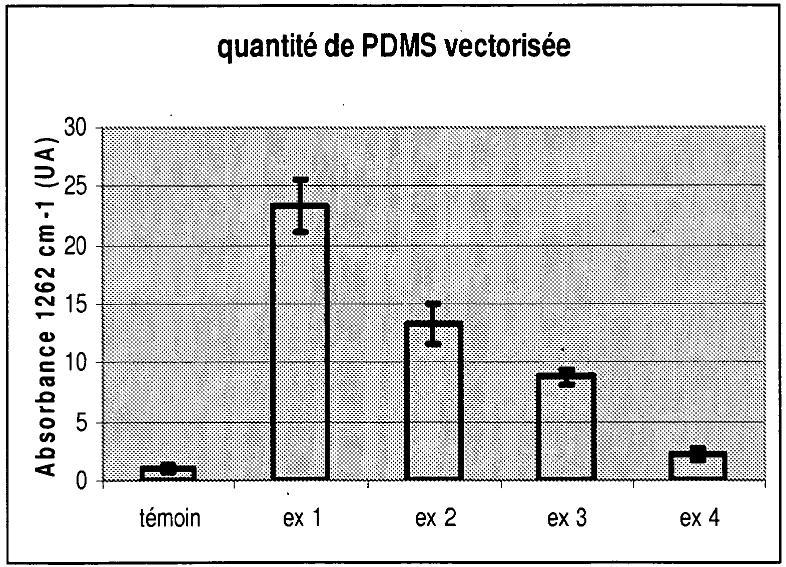

| | Témoin | **1** | **2** | **3** | **4** |
|---|---|---|---|---|---|
| **Quantité de PDMS (% MA) contenue dans le shampooing** | 2,6% | 2,6% | 1% | 0,5% | 0,1% |
| Quantité de PDMS (en U.A.) déposée sur les cheveux | **1** | **23,4** | **13,3** | **8,8** | **2,2** |

Comme le montre les résultats précédents, le shampooing selon l'invention (ex 1) permet de vectoriser plus de 20 fois la quantité de silicone déposée par le shampooing témoin à concentration équivalente en silicone dans les compositions (2,6% MA).

On note également qu'en diminuant dans les compositions selon l'invention (ex 4) la concentration en silicone de 26 fois (ex.4) comparativement à la composition témoin, la quantité de silicone vectorisée par la composition selon l'invention est encore deux fois plus importante que la quantité déposée par le shampooing témoin.

Les compositions selon l'invention permettent d'obtenir le même bénéfice cosmétique en utilisant beaucoup moins de silicone.

### EXEMPLE 5

On a réalisé la composition de shampooing conforme à l'invention suivante :

| | **5** |
|---|---|
| Coco glucoside [1] | 5 gMA |
| Coco amidopropyl bétaine [2] | 5,4 gMA |
| Lauryl ether (5 OE) carboxylate de sodium [3] | 3 gMA |
| Sodium lauryl ether sulfate de sodium [4] | 4 gMA |
| Polyquaternium 6 [5] | 0,8 gMA |
| Red 7 [7] | 1 |
| Conservateur | qs |
| Parfum | qs |
| Acide citrique | qs pH 6,5 |
| Eau | qsp 100 g |

| | |
|---|---|
| *[7] Pigment UNIPURE RED LC 3079 commercialisé par LCW* | |

1 gramme de la précédente formule est appliqué sur une mèche de cheveux naturels caucasiens blancs à 90% de 25cm de longueur, pesant 2,7g.

La mèche est malaxée 15 secondes, laissée posée 5 minutes puis rincée 10 secondes (eau 30°C / débit 30l/h) et enfin peignée. La mèche humide est ensuite séchée au casque 30 minutes à 70°C.

Le shampooing témoin est constitué par une composition contenant des tensioactifs détergents, un polymère cationique et une dimethicone auquel a été ajouté 1% MA du même pigment rouge que celui contenu dans la composition selon l'invention. Le shampooing témoin est appliqué dans les mêmes conditions que la composition selon l'invention.

La coloration des mèches de cheveux traitées avec la composition de l'invention est beaucoup plus importante à concentration équivalente en pigment que celle des cheveux traités avec le shampooing témoin.

On a noté également que le transfert de pigments sur la peau avec la composition de l'invention est faible malgré la grande quantité de pigment déposée sur la fibre kératinique.

## Revendications

1. Composition cosmétique détergente, **caractérisée par le fait qu'**elle comprend, dans un milieu aqueux cosmétiquement acceptable, (A) au moins un tensioactif anionique comprenant au moins un groupe choisi parmi sulfate, sulfonate, (B) au moins un tensioactif anionique carboxylique différent du tensioactif cité en (A), (C) au moins un tensioactif amphotère, (D) au moins un tensioactif non ionique alkylpolyglycoside, (E) au moins un polymère cationique ayant une densité de charge cationique supérieure à 4 meq/g et (F) au moins un agent bénéfique des matières kératiniques différent de (E),
le rapport en poids tensioactif anionique sulfate ou sulfonate / tensioactif amphotère allant de 0,1 à 2.

2. Composition selon la revendication 1, **caractérisée par le fait que** la quantité totale de tensioactif va de 4 % à 50 % en poids par rapport au poids total de la composition, de préférence de 6 % à 35 % en poids et plus préférentiellement de 8 % à 25 % en poids.

3. Composition selon l'une quelconque des revendications 1 ou 2, **caractérisée en ce que** le(s) tensioactif(s) anionique(s) sulfate ou sulfonate est (sont) présent(s) dans des concentrations allant de 1 à 50 % en poids, de préférence de 2 à 25 % en poids, par rapport au poids total de la composition.

4. Composition selon l'une quelconque des revendications 1 à 3, **caractérisée en ce que** ledit(s) tensioactif(s) anionique(s) carboxylique(s) est(sont) présent(s) dans des concentrations allant de 0,5 à 15 % en poids, de préférence de 1 à 10 % en poids, et encore plus particulièrement de 1,5 à 8% en poids par rapport au poids total de la composition.

5. Composition selon l'une quelconque des revendications 1 à 4, **caractérisée en ce que** le(s) tensioactif(s) amphotère(s) est(sont) présent(s) dans des concentrations allant de 0,1 à 20 % en poids, de préférence de 1 à 15 % en poids, plus particulièrement 2 à 10% en poids par rapport au poids total de la composition.

6. Composition selon l'une quelconque des revendications 1 à 5, **caractérisée en ce que** ledit(s) tensioactif(s) non ionique(s) alkylpolyglycoside est(sont) présent(s) dans des concentrations allant de 0,1 à 20 % en poids, de préférence de 1 à 15 % en poids, par rapport au poids total de la composition.

7. Composition selon l'une quelconque des revendications 1 à 6, **caractérisée par le fait que** le rapport en poids tensioactif anionique sulfate ou sulfonate /tensioactif anionique carboxylique va de 0,1 à 10 et de préférence de 0,5 à 5.

8. Composition selon l'une quelconque des revendications 1 à 8, **caractérisée par le fait que** le rapport en poids tensioactif anionique carboxylique/ tensioactif amphotère va de 0,1 à 10, de préférence de 0,2 à 5 et encore plus préférentiellement de 0,3 à 2.

9. Composition selon l'une quelconque des revendications 1 à 8, **caractérisé en ce que** les alkylpolyglucosides sont représenté par la formule générale suivante :
R₁O-(R₂O)ₜ (G)ᵥ (4)
dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un sucre comportant de 5 à 6 atomes de carbone, t désigne une valeur allant de 0 à 10, de préférence 0 à 4, et v désigne une valeur allant de 1 à 15.

10. Composition selon l'une quelconque des revendications 1 à 9, **caractérisée par le fait que** lesdits agents bénéfiques sont choisis parmi ;
(1) les saccharides, les oligosaccharides, les polysaccharides hydrolysés ou non, modifiés ou non,
(2) les acides aminés, les oligopeptides, les peptides, les protéines hydrolysées ou non, modifiées ou non.
(3) les acides et alcools gras ramifiés ou non,
(4) les cires animales, végétales ou minérales,
(5) les céramides et les pseudo-céramides,
(6) les acides organiques hydroxylés,
(7) les filtres UV,
(8) les antioxydants et les agents anti-radicaux libres,
(9) les chélatants,
(10) les agents antipelliculaires,
(11) les agents régulateurs de séborrée,
(12) les agents apaisants,
(13) les tensioactifs cationiques,
(14) les polymères cationiques ayant une densité de charge cationique inférieure ou égal à 4meq/g,
(15) les silicones,
(16) les huiles minérales, végétales ou animales,
(17) les polyisobutènes et poly(alpha-oléfines),
(18) les esters,
(19) les polymères anioniques solubles ou dispersés,
(20) les polymères non-ioniques solubles ou dispersés,
et leurs mélanges.

11. Composition selon l'une quelconque des revendications 1 à 10, **caractérisée par le fait que** lesdits agents bénéfiques sont choisis parmi les silicones.

12. Composition selon l'une quelconque des revendications 1 à 11, **caractérisée par le fait que** lesdits agents bénéfiques sont présents dans des concentrations allant de 0,001 à 20 %, et de préférence de 0,005 à 15 % en poids par rapport au poids total de la composition et encore plus particulièrement de 0,01 à 10% en poids.

13. Composition selon l'une quelconque des revendications 1 à 12, **caractérisée en ce que** ledit polymère cationique représente de 0,01 % à 10 % en poids, de préférence de 0,05 % à 8 % en poids, et encore plus préférentiellement de 0,1 % à 5 % en poids, du poids total de la composition.

14. Utilisation d'une composition telle définie dans l'une quelconque des revendications 1 à 13 pour le nettoyage et/ou le démaquillage des matières kératiniques.

15. Procédé de lavage et de conditionnement des matières kératiniques telles que les cheveux consistant à appliquer sur lesdites matières mouillées une quantité efficace d'une composition telle que définie dans l'une quelconque des revendications 1 à 13, puis à effectuer un rinçage à l'eau après un éventuel temps de pause.

## Claims

1. Detergent cosmetic composition, which comprises, in a cosmetically acceptable aqueous medium, (A) at least one anionic surfactant comprising at least one group chosen from sulfate, sulfonate, (B) at least one carboxylic anionic surfactant other than the surfactant mentioned in (A), (C) at least one amphoteric surfactant, (D) at least one alkylpolyglycoside nonionic surfactant, (E) at least one cationic polymer with a cationic charge density of greater than 4 meq./g and (F) at least one agent that is beneficial to keratin materials other than (E),
the sulfate or sulfonate anionic surfactant/amphoteric surfactant weight ratio ranging from 0.1 to 2.

2. Composition according to Claim 1, **characterized in that** the total amount of surfactant ranges from 4% to 50% by weight, preferably from 6% to 35% by weight and more preferentially from 8% to 25% by weight relative to the total weight of the composition.

3. Composition according to either of Claims 1 and 2, **characterized in that** the sulfate or sulfonate anionic surfactant(s) is (are) present in concentrations ranging from 1% to 50% by weight and preferable from 2% to 25% by weight relative to the total weight of the composition.

4. Composition according to any one of Claims 1 to 3, **characterized in that** said carboxylic anionic surfactant(s) is (are) present in concentrations ranging from 0.5% to 15% by weight, preferable from 1% to 10% by weight and even more particularly from 1.5% to 8% by weight relative to the total weight of the composition.

5. Composition according to any one of Claims 1 to 4, **characterized in that** the amphoteric surfactant(s) is (are) present in concentrations ranging from 0.1% to 20% by weight, preferable from 1% to 15% by weight and more particularly from 2% to 10% by weight relative to the total weight of the composition.

6. Composition according to any one of Claims 1 to 5, **characterized in that** said alkylpolyglycoside nonionic surfactant(s) is (are) present in concentrations ranging from 0.1% to 20% by weight and preferably from 1% to 15% by weight relative to the total weight of the composition.

7. Composition according to any one of Claims 1 to 6, **characterized in that** the sulfate or sulfonate anionic surfactant/carboxylic anionic surfactant weight ratio ranges from 0.1 to 10 and preferably from 0.5 to 5.

8. Composition according to any one of Claims 1 to 7, **characterized in that** the carboxylic anionic surfactant/amphoteric surfactant weight ratio ranges from 0.1 to 10, preferable from 0.2 to 5 and even more preferentially from 0.3 to 2.

9. Composition according to any one of Claims 1 to 8, **characterized in that** the alkylpolyglucosides are represented by the following general formula:
R₁O-(R₂O)ₜ(G)ᵥ (4)
in which R₁ represents a linear or branched alkyl and/or alkenyl radical containing from about 8 to 24 carbon atoms, an alkylphenyl radical in which the linear or branched alkyl radical contains from 8 to 24 carbon atoms, R₂ represents an alkylene radical containing from about 2 to 4 carbon atoms, G represents a sugar unit containing 5 or 6 carbon atoms, t denotes a value ranging from 0 to 10 and preferably from 0 to 4, and v denotes a value ranging from 1 to 15.

10. Composition according to any one of Claims 1 to 9, **characterized in that** said beneficial agents are chosen from:
(1) hydrolyzed or nonhydrolyzed, modified or unmodified saccharides, oligosaccharides and polysaccharides,
(2) hydrolyzed or nonhydrolyzed, modified or unmodified amino acids, oligopeptides, peptides and proteins,
(3) branched or unbranched fatty acids and alcohols,
(4) animal, plant or mineral waxes,
(5) ceramides and pseudoceramides,
(6) hydroxylated organic acids,
(7) UV-screening agents,
(8) antioxidants and free-radical scavengers,
(9) chelating agents,
(10) antidandruff agents,
(11) seborrhea regulators,
(12) calmatives,
(13) cationic surfactants,
(14) cationic polymers with a cationic charge density of less than or equal to 4 meq./g,
(15) silicoses,
(16) mineral, plant or animal oils,
(17) polyisobutenes and poly(alpha-olefins),
(18) esters,
(19) soluble or dispersed anionic polymers,
(20) soluble or dispersed nonionic polymers,
and mixtures thereof.

11. Composition according to any one of Claims 1 to 10, **characterized in that** said beneficial agents are chosen from silicones.

12. Composition according to any one of Claims 1 to 11, **characterized in that** said beneficial agents are present in concentrations ranging from 0.001% to 20%, preferable from 0.005%- to 15% and even more particularly from 0.01% to 10% by weight relative to the total weight of the composition.

13. Composition according to any one of Claims 1 to 12, **characterized in that** said cationic polymer represents from 0.01% to 10% by weight, preferably from 0.05% to 8% by weight and even more preferentially from 0.1% to 5% by weight relative to the total weight of the composition.

14. Use of a composition as defined in any one of Claims 1 to 13 for cleansing and/or removing makeup from keratin materials.

15. Process for washing and conditioning keratin materials such as the hair, which consists in applying to said wet materials an effective amount of a composition as defined in any one of Claims 1 to 13, and then in rinsing them with water after an optional leave-in time.

## Patentansprüche

1. Kosmetische Reinigungszusammensetzung, **dadurch gekennzeichnet, dass** sie in einem kosmetisch unbedenklichen wässrigen Medium (A) mindestens ein anionisches Tensid mit mindestens einer aus Sulfat und Sulfonat ausgewählten Gruppe, (B) mindestens ein carboxylgruppenhaltiges anionisches Tensid, das von dem in (A) aufgeführten Tensid verschieden ist, (C) mindestens ein amphoteres Tensid, (D) mindestens ein nichtionisches Alkylpolyglucosid-Tensid, (E) mindestens ein kationisches Polymer mit einer kationischen Ladungsdichte von mehr als 4 meq/g und (F) mindestens ein Vorteilsmittel für Keratinmaterialien, das von (E) verschieden ist, umfasst,
wobei das Gewichtsverhältnis von anionischem Sulfat- oder Sulfonat-Tensid zu amphoterem Tensid im Bereich von 0,1 bis 2 liegt.

2. Zusammensetzung nach Anspruch 1, **dadurch gekennzeichnet, dass** die Gesamtmenge an Tensid 4 bis 50 Gew.-%, bezogen auf das Gesamtgewicht der Zusammensetzung, vorzugsweise 6 bis 35 Ges.-% und weiter bevorzugt 8 bis 25 Ges.-% beträgt.

3. Zusammensetzung nach einem der Ansprüche 1 oder 2, **dadurch gekennzeichnet, dass** das anionische Sulfat- oder Sulfonat-Tensid bzw. die anionischen Sulfat- oder Sulfonat-Tenside in Konzentrationen im Bereich von 1 bis 50 Ges.-%, vorzugsweise 2 bis 25 Ges.-%, bezogen auf das Gesamtgewicht der Zusammensetzung, vorliegt bzw. vorliegen.

4. Zusammensetzung nach einem der Ansprüche 1 bis 3, **dadurch gekennzeichnet, dass** das carboxylgruppenhaltige anionische Tensid bzw. die carbonylgruppenhaltigen anionischen Tenside in Konzentrationen im Bereich von 0,5 bis 15 Gew.-%, verzugsweise 1 bis 10 Gew.-%, spezieller 1,5 bis 8 Gew.-%, bezogen auf das Gesamtgewicht der Zusammensetzung, vorliegt bzw. vorliegen.

5. Zusammensetzung nach einem der Ansprüche 1 bis 4, **dadurch gekennzeichnet, dass** das amphotere Tensid bzw. die amphoteren Tenside in Konzentrationen im Bereich von 0,1 bis 20 Ges.-%, vorzugsweise 1 bis 15 Gew.-%, spezieller 2 bis 10 Ges.-%, bezogen auf das Gesamtgewicht der Zusammensetzung, vorliegt bzw. vorliegen.

6. Zusammensetzung nach einem der Ansprüche 1 bis 5, **dadurch gekennzeichnet, dass** das nichtionische Alkylpolyglucosid-Tensid bzw. die nichtionischen Alkylpolyglucosid-Tenside in Konzentrationen im Bereich von 0,1 bis 20 Gew.-%, vorzugsweise 1 bis 15 Ges.-%, bezogen auf das Gesamtgewicht der Zusammensetzung, vorliegt bzw. vorliegen.

7. Zusammensetzung nach einem der Ansprüche 1 bis 6, **dadurch gekennzeichnet, dass** das Gewichtsverhältnis von anionischem Sulfat- oder Sulfonat-Tenside zu carboxylgruppenhaltigem anionischem Tensid im Bereich von 0,1 bis 10 und vorzugsweise 0,5 bis 5 liegt.

8. Zusammensetzung nach einem der Ansprüche 1 bis 7, **dadurch gekennzeichnet, dass** das Gewichtsverhältnis von carboxylgruppenhaltigem anionischem Tensid zu amphoteren Tensid im Bereich von 0,1 bis 10, vorzugsweise 0,2 bis 5 und noch weiter bevorzugt 0,3 bis 2 liegt.

9. Zusammensetzung nach einem der Ansprüche 1 bis 8, **dadurch gekennzeichnet, dass** die Alkylpolyglucoside durch die folgende allgemeine Formel wiedergegeben werden:
R₁O-(R₂O)ₜ(G)ᵥ (4)
worin R₁ für einen linearem oder verzweigten Alkyl- und/oder Alkinylrest mit etwa 8 bis 24 Kohlenstoffatomen oder einen Alkylphenylrest, dessen linearer oder verzweigter Alkylrest 8 bis 24 Kohlenstoffatome enthält, steht, R₂ für einen Alkylenrest mit etwa 2 bis 4 Kohlenstoffatomen steht, G für einen Zucker mit 5 bis 6 Kohlenstoffatomen steht, t für einen Wert im Bereich von 0 bis 10, vorzugsweise 0 bis 4, steht und v für einen Wert im Bereich von 1 bis 15 steht.

10. Zusammensetzung nach einem der Ansprüche 1 bis 9, **dadurch gekennzeichnet, dass** die Vorteilsmittel ausgewählt sind aus:
(1) hydrolysierten oder nicht hydrolysierten, modifizierten oder nicht modifizierten Sacchariden, oligosacchariden und Polysacchariden,
(2) hydrolysierten oder nicht hydrolysierten, modifizierten oder nicht modifizierten Aminosäuren, Oligopeptiden und Peptiden,
(3) verzweigten oder unverzweigten Fettsäuren und Fettalkoholen,
(4) tierischen, pflanzlichen oder mineralischen Wachsen,
(5) Ceramiden und Pseudoceramiden,
(6) organischen Hydroxysäuren,
(7) UV-Schutzmitteln,
(8) Antioxidanzien und Radikalfängern,
(9) Chelatbildnern,
(10) Antischuppenmitteln,
(11) Seborrhoe regulierenden Mitteln,
(12) beruhigenden Mitteln,
(13) kationischen Tensiden,
(14) kationischen Polymeren mit einer kationischen Ladungsdichte kleiner gleich 4 meq/g,
(15) Silikonen,
(16) mineralischen, pflanzlichen oder tierischen Ölen,
(17) Polyisobutenen und Poly(alpha-olefinen),
(18) Estern,
(19) löslichen oder dispergierten anionischen Polymeren,
(20) löslichen oder dispergierten nichtionischen Polymeren
und Mischungen davon.

11. Zusammensetzung nach einem der Ansprüche 1 bis 10, **dadurch gekennzeichnet, dass** die Vorteilsmittel aus Silikonen ausgewählt sind.

12. Zusammensetzung nach einem der Ansprüche 1 bis 11, **dadurch gekennzeichnet, dass** die Vorteilsmittel in Konzentrationen im Bereich von 0,001 bis 20 Gew.-% und vorzugsweise 0,005 bis 15 Gew.-%, bezogen auf das Gesamtgewicht der Zusammensetzung, und noch weiter bevorzugt 0,01 bis 10 Gew.-%, vorliegen.

13. Zusammensetzung nach einem der Ansprüche 1 bis 12, **dadurch gekennzeichnet, dass** das kationische Polymer 0,01 bis 10 Ges.-%, vorzugsweise 0,05 bis 8 Ges.-% und noch weiter bevorzugt 0,1 bis 5 Gew.-% des Gesamtgewichts der Zusammensetzung ausmacht.

14. Verwendung einer Zusammensetzung gemäß einem der Ansprüche 1 bis 13 zum Reinigen und/oder Abschminken von Keratinmaterialien.

15. Verfahren zum Waschen und Konditionieren von Keratinmaterialien wie den Haaren, das darin besteht, dass man auf die feuchten Materialien eine wirksame Menge einer Zusammensetzung gemäß einem der Ansprüche 1 bis 13 aufbringt und danach mit Wasser spült, gegebenenfalls nach einer Wartezeit.
